# EUROPEAN PATENT SPECIFICATION

(11) **EP 1 383 542 B1**
(45) Date of publication and mention of the grant of the patent: **02.04.2008**
(21) Application number: 02727579.1
(22) Date of filing: 23.04.2002
(51) Int. Cl.: A61K 49/00, G01N 33/52, G01N 33/50

(54) **IN VIVO METHOD FOR MEASURING BINDING OF CHEMICAL ACTIVES TO SKIN OR SPECIFIC CONSTITUENTS OF SKIN**
IN-VIVO VERFAHREN ZUR MESSUNG VON AUF DER HAUT ODER DESSEN KOMPONENTEN BINDENDEN AKTIVEN CHEMISCHEN SUBSTANZEN
PROCEDE IN VIVO DE MESURE DE LIAISON DE COMPOSANTS ACTIFS CHIMIQUES DE LA PEAU OU DE CONSTITUANTS SPECIFIQUES DE LA PEAU

(30) Priority: 30.04.2001 US 287889 P
(43) Date of publication of application: 28.01.2004
(73) Proprietor: UNILEVER PLC, London, Greater London EC4P 4BQ (GB); UNILEVER N.V., 3013 AL Rotterdam (NL)
(72) Inventor: LEESON, Daniel Thorn, Edgewater, NJ 07020 (US); SUBRAMANYAN, Krishna Kumar, Edgewater, NJ 07020 (US); ANANTHAPADMANABHAN, Kavssery Parameswaran, Edgewater, NJ 07020 (US)
(74) Representative: Mulder, Cornelis Willem Reinier
(86) International application number: PCT/EP2002/004517
(87) International publication number: WO 2002/087628

(56) References cited:
- WO-A-92/15700
- DEL POZO A ET AL: "Aplicacion de una tecnica colorimetrica para la evaluacion del efecto de preparados de base tensiactiva sobre el estrato corneo." CIENCIA Y TECNOLOGIA PHARMACEUTICA, vol. 11, no. 3, 2001, pages 140-144, XP001095238 ISSN: 1575-3409
- SAUERMANN G ET AL: "COMPARATIVE STUDY OF SKIN CARE EFFICACY AND IN-USE PROPERTIES OF SOAP AND SURFACTANT BARS" JOURNAL OF THE SOCIETY OF COSMETIC CHEMISTS, vol. 37, no. 5, 1986, pages 309-327, XP001094733 ISSN: 0037-9832
- PAGNONI ALESSANDRA ET AL: ""Pyranine, a fluorescent dye, detects subclinical injury to sodium lauryl sulfate"" JOURNAL OF COSMETICAL SCIENCE, vol. 49, January 1998 (1998-01), pages 33-38, XP001095530 cited in the application
- IMOKAWA G ET AL.: "CUMULATIVE EFFECTS OF SURFACTANTS ON CUTANEOUS HORNY LAYERS: ADSORPTION ONTO HUMAN KERATIN LAYERS IN VIVO" CONTACT DERMATITIS , vol. 5, no. 6, 1979, pages 357-366, XP001095284 cited in the application
- STAMATAS GEORGIOS N ET AL: "Non-invasive method for quantitative evaluation of exogenous compound deposition on skin." THE JOURNAL OF INVESTIGATIVE DERMATOLOGY. UNITED STATES FEB 2002, vol. 118, no. 2, February 2002 (2002-02), pages 295-302, XP001095236 ISSN: 0022-202X
- PAYE MARC ET AL: "Dansyl chloride labelling of stratum corneum: Its rapid extraction from skin can predict skin irritation due to surfactants and cleansing products." CONTACT DERMATITIS, vol. 30, no. 2, 1994, pages 91-96, XP001095239 ISSN: 0105-1873

## Description

The present invention relates to *in vivo* methods for measuring the binding of chemical actives, more specifically surfactants, to skin, and more specifically to proteins in skin.

*In vivo* and *ex vivo* methods for measuring the binding of chemicals, mainly surfactants, to skin are known. These methods typically involve applying a strongly absorbing or luminescent probe/marker either before or after treatment with a solution of the chemical compound or mixture of compounds in question, and subsequently measuring the intensity of the absorbance or luminescence on skin.

In "Cumulative effect of surfactants on cutaneous horny layers: Adsorption onto human keratin layers in vivo", G. Imokawa and Y. Mishima, Contact Dermatitis 5(6) pp 357-366 (1979), it is suggested that the absorbance of the probe/marker applied after treatment is a measure for the amount of binding by surfactants. It is argued that when surfactants bind to the skin during treatment they block potential binding sites for the probe/marker. Hence fewer probe probe/marker molecules bind to the skin resulting in a less intense color than is observed when the probe/marker is applied without treatment. However, the authors are not specific as to what the specific binding sites are, i.e. what constituents of skin the probe/marker binds to.

In "Interactions of cleansing bars with stratum corneum proteins: An in vitro fluorescence spectroscopic study", S. Mukherjee et al., J. Soc. Cosmet. Chem. 76 (1995) pp 301-320, a protein-binding fluorescent probe/marker is applied to human and porcine skin *ex vivo* before treatment with various surfactant solutions. Subsequently, the fluorescence spectrum of the probe/marker is taken, and the fractional displacement of the probe/marker by the surfactants is calculated by taking the ratio of the fluorescence intensity of the probe/marker to that of the tryptophan residues of the skin proteins.

However, no changes in the spectral shape of the fluorescence spectrum of the probe/marker itself are observed. The method of the invention is not disclosed.

In "Pyranine, a fluorescent dye, detects subclinical injury to sodium lauryl sulfate", A. Pagnoni et al., J. Cosmet. Sci. 49 (1998) pp 33-38, a fluorescent probe/marker, 8-hydroxyl-1,3,'6-pyrene-trisulfonic acid (pyranine) is applied in *vivo* after treatment. It is observed that when skin is exposed to sodium lauryl sulfate (SLS), an anionic surfactant, for 24 hours, a much stronger fluorescence is observed than when the probe is applied without any prior treatment.

According to the argumentation used in the previously mentioned work, binding of SLS to the skin should lead to a decrease in the fluorescence. The fact that the fluorescence increases rather than decreases suggests that the 24-hour treatment reduces the barrier function of the skin, thus exposing more binding sites for the fluorescent probe/marker. This is supported by transepidermal water loss measurements.

Del Pozo et al., in the Ciencia Y Technologia Pharmaceutica, (11 (3) (2001) pp 140 -. 144), discloses an in-vivo method for measuring the mildness/harshness of a surfactant by first applying the surfactant in question to the skin, followed by treatment with pyranine, the by visualising and measuring the fluorescence by irradiation at 365 nm.

Sauermann et al., in the Journal Soc. Cosmet. Chem. (37(5), (1986) pp. 309-327), discloses an in vivo method for assessing the irritancy of soaps by measuring the absorbance of a cationic dye after application of surfactants or soap bars.

Pagoni et al, in the Journal of investigative dermatology, (108(4), (1997), pp 674), discloses the use of pyranine as fluorescent dye in-vivo, for evaluating skin damage with detergents. The skin damage is visualised by taking fluorescent photographs at a specified site.

The present invention relates to an *in vivo* method for measuring the binding of chemical compounds, specifically surfactants, and more specifically surfactants from cleansers, to skin, and more specifically to proteins in skin.

The present invention discloses one specific embodiment for an *in vivo* measurement.

In the embodiment of the present invention, the amount of surfactant binding to the skin after application of a cleansing product is measured by choosing a desired spot oh the subject's body; washing the skin with the product in question; applying a desired amount of a fluorescent probe/marker (e.g. pyranine) and obtaining a fluorescence spectrum of the area the fluorescent or absorbing probe was applied to, e.g. by using a fiber optic probe/marker; and calculating the ratio of the fluorescence intensity or absorbance of the probe/marker on skin at two different wavelengths.

More specifically, the invention comprises an *in vivo* method for measuring the binding affinity of chemicals, more specifically surfactants, which can be either pure surfactants or surfactants from cleanser formulations, to skin, and more specifically to proteins in skin. The method comprises:
1. choosing a desired site, typically 5.08cm x 10.16cm (2"x4") but the site can be either smaller or larger, on the body of a subject;
2. exposing the body site to the chemical or formulation of interest. Exposure to the chemical can be done in any desired way. In the case of surfactants or cleanser formulations, exposure typically involves washing the skin with the surfactant or cleanser formulation;
3. applying the probe/marker to an area within the site that was exposed to the chemical formulation, typically a circular area of 1.27 cm (0.5" diameter, but the area can be any shape or size provided it fully overlaps with the site that was treated in step 2;
4. acquiring a luminescence or absorbance spectrum of the area of the skin that the probe/marker was applied to;
5. determining the binding affinity of the chemical compound to a specific constituent of skin, e.g. skin protein, e.g., by calculating the ratio of the fluorescence intensity or absorbance at two different wavelengths, or by measuring any change in the spectrum of the probe/marker other than its amplitude.

The invention will now be described by way of example only, with reference to the accompanying drawings, in which:
- Figure 1 depicts the fluorescence spectra of pyranine in 50 mM Hepes buffer containing 2.5 mg/ml BSA (dashed line); on human skin when applied topically (dotted line);
- Figure 2 depicts the fluorescence spectra of pyranine in 50 mM Hepes buffer (solid line); in 50 mM Hepes buffer containing 2.5 mg/ml BSA (dashed line); and in 50 mM Hepes buffer containing 2.5 mg/ml BSA and 4x10⁻⁵ weight fraction SDS (dotted line);
- Figure 3 depicts fluorescence spectra of pyranine when topically applied to skin without any prior treatment (solid line) and when applied after a two-minute wash with a harsh soap bar (dashed line);
- Figure 4 depicts the fluorescence spectra of pyranine when topically applied to skin after washing with a mild syndet bar (solid line) and after washing with a harsh soap bar (dashed line); and
- Figure 5 depicts the results of a pilot clinical study. Displayed is the difference between the baseline fluorescence spectrum (no treatment) and the spectrum after a soap wash, as quantified by the difference in the ratio of the maximum fluorescence intensity and the intensity at 460 nm. Each bar represents the number of subjects.

The present invention relates to an *in vivo* method for measuring the ability of chemicals, more specifically surfactants, and even more specifically, surfactants applied from cleanser formulations, to bind to specific constituents of skin, e.g. proteins or lipids. The method is also applicable *ex vivo*. The method can be used as a quick and easy assay to determine the mildness of cleanser formulations.

Specifically, the invention provides a method for measuring the ability of chemicals to bind to proteins in skin *in vivo* comprising:
1. choosing a desired site, typically 2"x2", on the body of a subject;
2. exposing said site to the chemical compound in question, in the case of surfactants or cleanser formulations typically by washing the site, for any desired amount of time;
3. applying the luminescent or absorbing probe/marker, e.g., pyranine or any other probe/marker that exhibits a different luminescence or absorbance spectrum depending on its microenvironment within the skin, dissolved, typically in ethanol or water to typically a circular area approximately 1.26 cm (0.5") in diameter;
4. acquiring a luminescence or absorbance spectrum of the area of the skin that the probe/marker was applied to, e.g. by using a fiber optic probe/marker;
5. determining the binding affinity of the chemical compound to a specific constituent of skin, e.g. skin protein, by calculating the ratio of the fluorescence intensity or absorbance at two different wavelengths or by measuring any change in the spectrum of the probe/marker other than its amplitude.

Each of the process steps is discussed in more detail below and in the examples.

As noted, the first step in the *in vivo* method of determining the binding affinity of chemical compounds to specific constituents of skin according to the subject invention is to choose a desired spot on the subject suitable for measurements.

The method is applicable to any body site that can be treated with the chemical compound, the binding of which is to be measured. Restrictions in the size of the site to be chosen are only determined by the method of exposure to the chemical compound. For example, if the compound in question is a surfactant and it is applied through washing the body site with the surfactant, it would not be practical to choose an area smaller than roughly 1.26cm x 1.26cm (0.5"x0.5") in size. However, if the method of exposure allows it, a body site of much smaller dimensions can be chosen.

There is no upper limit on the size of the body site to be chosen. For example, if one desires to measure the binding of a cleanser formulation to skin, the whole body of the subject can be washed, and subsequently the binding can be measured at different sites on the body (see the second step).

In the second step of the invention, the body site that was chosen in the first step, is exposed to the chemical compound or mixture of compounds, the binding of which is to be measured.

There are no restrictions in the method by which the chemical compound is exposed to the skin or the duration of exposure other that it can be reasonably applied *in vivo.* If the method is applied *ex vivo*, there are no restrictions in the method or duration of exposure. Typical methods of exposure can be soaking the skin with the compound or mixture of compounds of interest, rubbing the skin with the compound, and particularly in the case of surfactants or cleanser formulations, washing the skin.

In the third step of the invention the luminescent or optically absorbing probe/marker is applied to the area that was exposed to the chemical compound(s) in the second step.

The probe/marker that is applied can be any probe/marker that exhibits differences in its luminescence or absorbance spectrum, other than the amplitude, depending on its microenvironment within the skin. Pyranine is a fluorescent probe/marker that can fluoresce both from a protonated and a deprotonated state. Each state fluoresces at different wavelengths. The fluorescence from the protonated state peaks between 930 nm and 460 nm, while the fluorescence from the deprotonated state peaks at 510 nm. The ratio of the fluorescence intensities from the two different states is strongly dependent on the micro-environment of the molecule.

For instance, the fluorescence spectrum of pyranine dramatically changes when it is bound to proteins as is illustrated in Example 1. This makes pyranine a preferred probe/marker to measure the binding of chemical compounds to skin proteins as is illustrated in Example 3. Different probes/markers may be used to measure binding of chemicals to constituents of skin other than proteins. For example, a probe/marker that exhibits a change in its luminescence or absorbance spectrum when it is in a lipid environment, would be a candidate to measure the binding of chemicals to skin lipids. *In vitro* luminescence or absorption spectra may be taken to determine the preferred micro-environment of a probe/marker (see Example 2).

There are no restrictions in the method by which the probe/marker is applied to the skin or the duration of application, other that it can be reasonably applied *in vivo*. If the method is applied *ex vivo,* the are no restrictions in the method or duration of exposure. Typical would be to topically apply the probe from solution, e.g. dissolved in alcohol or water, to a circular area of approximately 1.26 cm (0.5") in diameter. There are no restrictions to the size of the area that the probe/marker is applied to other than that it should fall within the area that was exposed to the chemical compounds in the second step. In some cases it may be desirable to expose a relatively large area in the second step, for instance by washing the whole forearm, and then subsequently apply the probe/marker to several smaller areas within the larger area that was exposed to the chemical compound.

In the fourth step of the invention a luminescence or absorbance spectrum is obtained of the probe/marker on skin. Typically these spectra are taken with a fluorometer (for fluorescent probe/markers) or a spectrophotometer (for absorbing probe/markers). It is important that the area of the skin that is covered by the measurement is equal to or smaller than the area of the skin that the probe/marker was applied to in the third step. This can be typically achieved by using a fiber optic probe/marker to illuminate the skin with the excitation light and to collect the resulting fluorescence light (for fluorescent probe/marker) or by applying the incident light and collecting the reflected light (for absorbing probe/marker).

In the fifth step of the invention the spectrum that is measured in the fourth step is analyzed to determine the binding of the chemical compound or mixture of compounds that was applied in the second step. For example, this can be done by taking the ratio of the fluorescence intensity or absorbance at two different wavelengths in the spectrum or by determining the wavelength of maximum fluorescence intensity or absorbance.

Except in the operating and comparative examples, or where otherwise explicitly indicated, all numbers in this description indicating amounts or ratios of materials or conditions or reaction, physical properties of materials and/or use are to be understood as modified by the word " about" .

Where used in the specification, the term "comprising" is intended to include the presence of stated features, integers, steps, components, but not to preclude the presence or addition of one or more features, integers, steps, components or groups thereof.

The following examples are intended to further illustrate the invention and are not intended to limit the invention in any way.

Unless indicated otherwise, all percentages are intended to be percentages by weight.

### METHODOLOGY

### Equipment.

Fluorescence spectra of pyranine in solutions and on skin were obtained using a Perkin Elmer LS50B fluorometer. In the case of spectra taken on skin, the fiber optic probe accessory was used.

### EXPERIMENTAL PROCEDURE

### In Vitro Experiments:

All spectra were taken at pyranine concentrations of 1.0 x10⁻⁸ M in 50 mM Hepes buffer (aqueous buffer solution with pH 6.7). Bovine Serum Albumim (BSA) concentrations were 2.5 mg/ml. Surfactant was added at weight fractions of 4x10⁻⁵.

### In Vivo Experiments

An area on the volar forearm, approximately 4"x2" in size, was washed with either a Dove® or an Ivory® soap bar for 2 minutes. The skin was subsequently rinsed with tap water for 10 seconds and subsequently patted dry. 100 µl of a 10,000 ppm solution of pyranine in ethanol was applied to a circular area of 1.26 cm (0.5") diameter at the center of the area that was washed. The fiber optic probe/marker was placed at the center of the circular area and the fluorescence spectrum was obtained.

### EXAMPLE 1

### Dependence of the Pyranine Fluorescence Spectrum on Micro-Environment

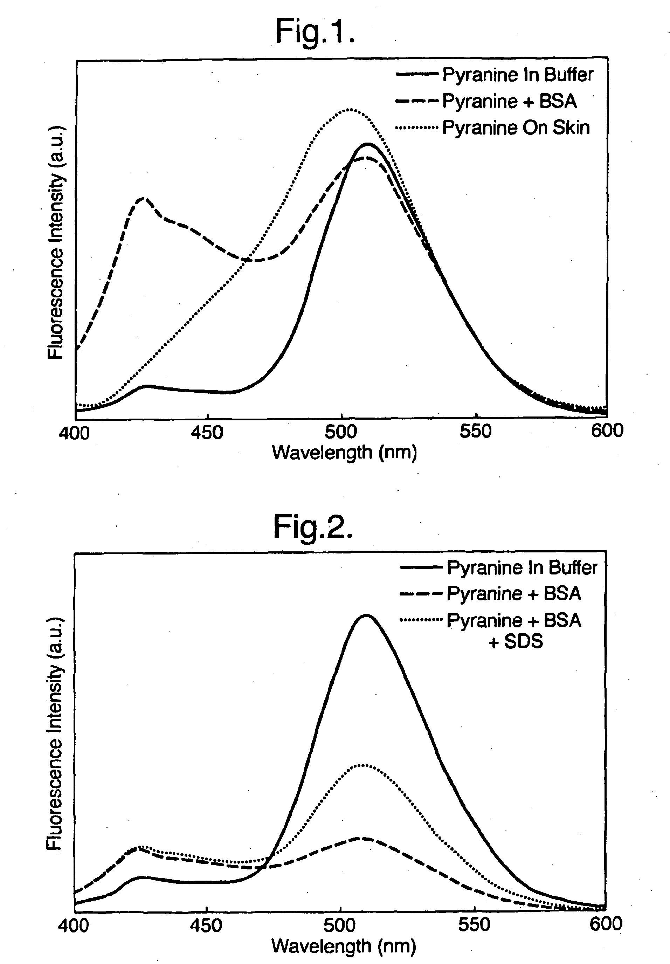

Figure 1 displays fluorescence spectra of pyranine in three different environments; dissolved in an aqueous buffer, dissolved in the same buffer with added protein (BSA), and on human skin, *in vivo.*

The spectrum in aqueous buffer shows an intense band, centered at around 510 nm. This band corresponds to the deprotonated state as was discussed earlier. In addition, a minor band around 425 nm can be observed, arising from the protonated state. When BSA is added the same bands can be observed in the spectrum, but with largely different relative intensities. The change in the relative intensities of the two bands results from binding of pyranine to the protein.

In this different micro-environment, fluorescence from the deprotonated form of pyranine is less favored than in free solution. The fluorescence spectrum of pyranine on skin also clearly exhibits multiple bands. The fluorescence from the protonated state is red-shifted relative to the free solution spectrum and clearly more intense. The fluorescence from the deprotonated state does not seem to have shifted significantly relative to the free solution spectrum. The higher relative intensity of the fluorescence from the protonated state suggests that topically applied pyranine binds to skin proteins.

### EXAMPLE 2

### The Effect of Surfactants on the Spectrum of Pyranine in Protein Solution

In order to test the applicability of pyranine fluorescence to monitor the binding of surfactants to proteins, the applicants studied the effect of added surfactant on the fluorescence spectrum of aqueous solutions of pyranine and BSA. Figure 2 shows how the addition of sodium dodecyl sulphate (SDS) results in a significant increase of the relative intensity of the fluorescence from the deprotonated state. This effect can be explained by the replacement of pyranine molecules bound to BSA by SDS molecules leading to an increased concentration of pyranine in free solution and therefore a relative increase in intensity of the fluorescence from the deprotonated state. The ratio of the fluorescence intensities from the deprotonated and protonated site is a measure of the ability of a surfactant to replace pyranine from BSA binding sites. Table 1 below shows this ratio for a number of surfactants when added to a pyranine/BSA solution at equal weight fractions.

It is clear from Table 1 that surfactants that are well known to be harsh to skin, such as SDS, have a higher ratio than known milder surfactants such as alkylpolyglycoside (APG) suggesting that the harshness/mildness of surfactants is, at least in part, determined by their affinity for protein binding sites.

**Table 1**

| System | \|509 nm\|426 nm |
|---|---|
| Pyranine | 8.03 |
| Pyranine + BSA + SDS | 2.23 |
| Pyranine + BSA + Sodium Laurate | 2.13 |
| Pyranine + BSA + Sodiumlauryl Ether Sulfate | 2.12 |
| Pyranine + BSA + Cocamilopropyl Betaine | 1.56 |
| Pyranine + BSA + APG | 1.34 |
| Pyranine + BSA | 1.17 |

### EXAMPLE 3

### The Effect of a Soap Wash on the Fluorescence Spectrum of Pyranine on Skin

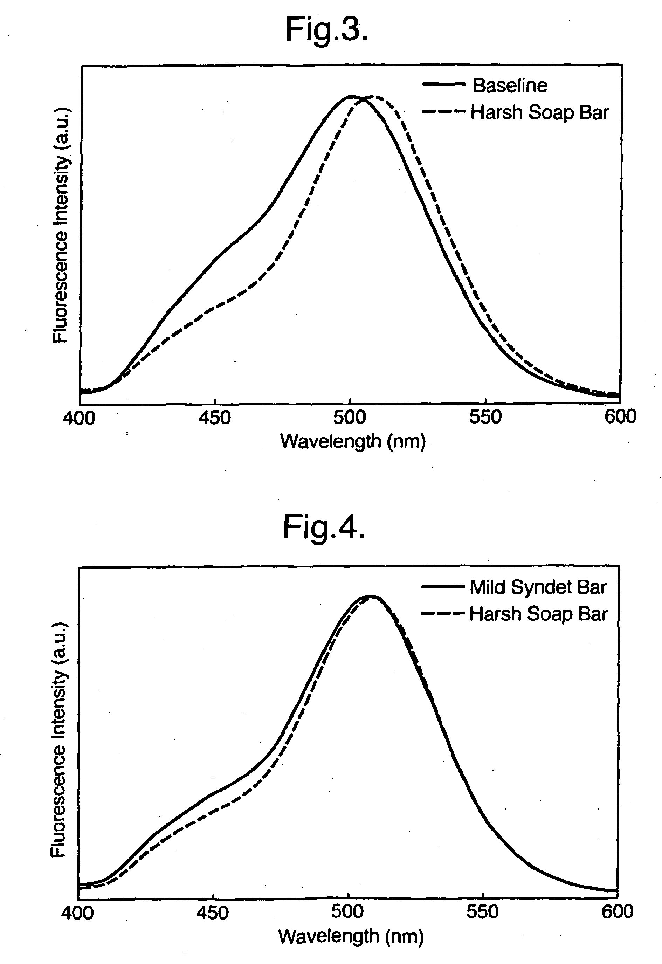

Figure 3 shows two fluorescence spectra of pyranine on skin, one where pyranine was applied without any prior treatment and one where pyranine was applied after a two-minute arm wash with a harsh soap bar. The soap treatment leads to a significant change in the fluorescence spectrum, most notably a reduced intensity of the band around 450 nm. The reduced intensity of the fluorescence from the protonated state is most likely due to the binding of surfactants from the soap bar to protein binding sites on skin.

When pyranine is applied after the soap wash some potential binding sites will already be occupied by surfactant molecules and therefore part of the probe molecules will bind superficially leading to a reduction of the fluorescence from the protonated state. This effect is significantly reduced when using a mild syndet bar as is shown in Figure 4, which shows spectra both after washing with a harsh soap bar and a mild syndet bar.

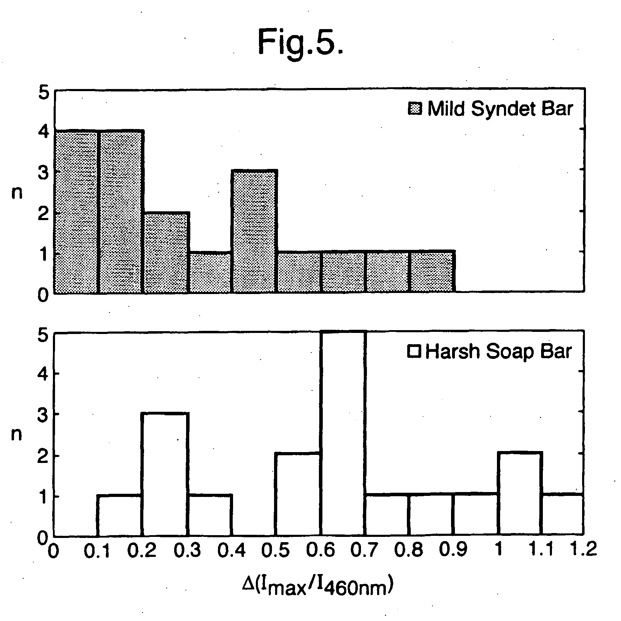

The effect of a soap wash on the fluorescence spectrum of pyranine on skin can be quantified by taking the ratio of the fluorescence intensity at peak height and the intensity at 460nm, and subsequently taking the difference between this ratio before and after washing with the soap bar. Figure 5 displays the results of a pilot clinical study clearly showing an overall stronger effect for the harsh soap bar.

## Claims

1. An *in vivo* method for measuring the binding of surfactants or mixtures of surfactant compounds to skin constituents comprising:
(a) choosing a desired site of any desired size on a subject's body;
(b) exposing said site, the binding of which is to be measured , to the surfactants or mixture of surfactant compounds by any desirable method;
(c) applying a luminescent or optically absorbing probe/marker to the area that was exposed to the surfactants or mixture of surfactant compounds;
(d) measuring the luminescence or absorbance spectrum of the probe/marker on skin; and
(e) determining the binding of the surfactant or surfactants the body site was exposed to from the luminescence or absorbance spectrum by measuring the change in the shape of the spectrum and subsequently quantifying said change in shape by calculating the ratio of the fluorescence intensity or absorbance at two different wavelengths.

2. A method according to Claim 1, wherein the skin constituent measured is a skin protein or proteins.

3. A method to measure mildness of a cleanser formulation which method comprises:
(a) choosing a desired skin site of desired size on a subject' s body;
(b) washing said skin with a surfactant or cleanser composition;
(c) applying luminescent or optically absorbing probe/marker to the area exposed to surfactant or cleansing composition;
(d) measuring the luminescence or absorbance spectrum of the probe/marker on skin; and
(e) determining the binding of surfactant or cleanser composition the body site was exposed to from the luminescence or absorbance spectrum by measuring the change in shape of the spectrum and subsequently quantifying said change in shape by calculating the ratio of the fluorescence intensity or absorbance at two different wavelengths.

## Patentansprüche

1. In-vivo-Verfahren zur Messung des Bindens von Tensiden oder Gemischen von Tensidverbindungen an Hautbestandteile, umfassend:
(a) Auswählen einer gewünschten Stelle von beliebiger gewünschter Größe auf dem Körper einer Person;
(b) Aussetzen der Stelle, an der das Binden zu messen ist, den Tensiden oder dem Gemisch von Tensidverbindungen durch ein beliebiges gewünschtes Verfahren;
(c) Auftragen einer lumineszierenden oder optisch absorbierenden Sonde / eines lumineszierenden oder optisch absorbierenden Markers auf die Fläche, die den Tensiden oder dem Gemisch von Tensidverbindungen ausgesetzt wurde;
(d) Messen der Lumineszenz oder des Absorptionsspektrums von der Sonde / dem Marker auf der Haut und
(e) Bestimmen des Bindens von dem Tensid oder Tensiden, dem/denen die Körperstelle ausgesetzt wurde, aus dem Lumineszenz- oder Absorptionsspektrum durch Messen der Änderung der Form des Spektrums und anschließend Quantifizieren der Änderung der Form durch Berechnen des Verhältnisses der Fluoreszenzintensität oder der Absorption bei zwei verschiedenen Wellenlängen.

2. Verfahren nach Anspruch 1, wobei der gemessene Hautbestandteil ein Hautprotein oder proteine ist.

3. Verfahren zum Messen der Mildheit von einer Reinigungsmittelformulierung, wobei das Verfahren umfasst:
(a) Auswählen der gewünschten Hautstelle von gewünschter Größe auf dem Körper einer Person;
(b) Waschen der Haut mit einem Tensid oder einer Reinigungsmittelzusammensetzung;
(c) Auftragen einer lumineszierenden oder optisch absorbierenden Sonde / eines lumineszierenden oder optisch absorbierenden Markers auf die dem Tensid oder reinigenden Zusammensetzung ausgesetzten Fläche;
(d) Messen des Lumineszenz- oder des Absorptionsspektrums von der Sonde / dem Marker auf der Haut und
(e) Bestimmen des Bindens von Tensid oder Reinigungsmittelzusammensetzung dem/der die Körperstelle ausgesetzt wurde, aus dem Lumineszenz- oder Absorptionsspektrum durch Messen der Änderung der Form des Spektrums und anschließend Quantifizieren der Änderung der Form durch Berechnen des Verhältnisses der Fluoreszenzintensität oder der Absorption bei zwei verschiedenen Wellenlängen.

## Revendications

1. Procédé in vivo pour mesurer la liaison de tensioactifs ou de mélanges de composés tensioactifs à des constituants de la peau, comprenant:
(a) le choix d'un site souhaité, d'une taille souhaitée quelconque, sur le corps d'un sujet ;
(b) l'exposition dudit site, dont la liaison doit être mesurée, aux tensioactifs ou au mélange de composés tensioactifs, par n'importe quel procédé souhaitable ;
(c) l'application d'une sonde/d'un marqueur luminescent ou optiquement absorbant à la zone qui a été exposée aux tensioactifs ou au mélange de composés tensioactifs ;
(d) la mesure du spectre de luminescence ou d'absorbance de la sonde/du marqueur sur la peau ; et
(e) la détermination de la liaison du tensioactif ou des tensioactifs auxquels le site du corps a été exposé à partir du spectre de luminescence ou d'absorbance par mesure du changement de la forme du spectre et ensuite la quantification dudit changement de forme par calcul du rapport de l'intensité de fluorescence ou de l'absorbance à deux longueurs d'onde différentes.

2. Procédé selon la revendication 1, dans lequel le constituant mesuré de la peau est une ou plusieurs protéines de la peau.

3. Procédé pour mesurer la douceur d'une formulation nettoyante, lequel procédé comprend:
(a) le choix d'un site de peau souhaité de taille souhaitée sur le corps d'un sujet ;
(b) le lavage de ladite peau avec un tensioactif ou une composition nettoyante ;
(c) l'application d'une sonde/d'un marqueur luminescent ou optiquement absorbant à la zone exposée au tensioactif ou à la composition nettoyante ;
(d) la mesure du spectre de luminescence ou d'absorbance de la sonde/du marqueur sur la peau ; et
(e) la détermination de la liaison du tensioactif ou de la composition nettoyante auquel le site du corps a été exposé à partir du spectre de luminescence ou d'absorbance par mesure du changement de la forme du spectre et ensuite la quantification dudit changement de forme par calcul du rapport de l'intensité de fluorescence ou de l'absorbance à deux longueurs d'onde différentes.
